# EUROPÄISCHE PATENTSCHRIFT

(11) **EP 1 362 231 B1**
(45) Veröffentlichungstag und Bekanntmachung des Hinweises auf die Patenterteilung: **29.03.2006**
(21) Anmeldenummer: 02704606.9
(22) Anmeldetag: 17.01.2002
(51) Int. Cl.: G01N 21/35, G01N 33/48

(54) **VERFAHREN ZUM NACHWEIS VON TSE-INDUZIERTEN VERÄNDERUNGEN IM MENSCHLICHEN UND TIERISCHEN KÖRPER**
METHOD FOR DETECTING TSE-INDUCED MODIFICATIONS IN THE HUMAN AND ANIMAL BODY
PROCEDE DE DETECTION DE MODIFICATIONS INDUITES PAR L'ENCEPHALOPATHIE SPONGIFORME CHEZ L'HOMME ET L'ANIMAL

(30) Priorität: 22.02.2001 DE 10109901
(43) Veröffentlichungstag der Anmeldung: 19.11.2003
(73) Patentinhaber: Bundesrepublik Deutschland Vertreten Durch Das Bundesministerium Für Gesundhait, Dieses Vertreten Durch Das Robert-Koch-Instit, Nordufer 20 (DE)
(72) Erfinder: NAUMANN, Dieter, 10997 Berlin (DE); BEEKES, Michael, 14612 Falkensee (DE); SCHMITT, Jürgen, 69118 Heidelberg (DE); UDELHOVEN, Thomas, 54296 Trier (DE); BRAUER, Angelika, 14502 Berlin (DE)
(74) Vertreter: Bösl, Raphael Konrad
(86) Internationale Anmeldenummer: PCT/DE2002/000210
(87) Internationale Veröffentlichungsnummer: WO 2002/066963

(56) Entgegenhaltungen:
- DE-C- 19 923 811
- US-A- 5 869 001
- US-A- 5 977 545
- KNEIPP J ET AL: "Detection of pathological molecular alterations in scrapie-infected hamster brain by Fourier transform infrared (FT-IR) spectroscopy" BIOCHIMICA ET BIOPHYSICA ACTA. MOLECULAR BASIS OF DISEASE, AMSTERDAM, NL, Bd. 1501, Nr. 2-3, 15. Juni 2000 (2000-06-15), Seiten 189-199, XP004276997 ISSN: 0925-4439

## Beschreibung

Die Erfindung betrifft ein Verfahren zum Nachweis von durch transmissible spongiforme Enzephalopathien (TSE) induzierten pathologischen Veränderungen im menschlichen und tierischen Körper.

Transmissible spongiforme Enzephalopathien sind übertragbare neurodegenerative Erkrankungen des Zentralnervensystems (ZNS) mit tödlichem Verlauf. Die Krankheit betrifft sowohl Säugetiere als auch den Menschen. TSE dient als Oberbegriff zur Bezeichnung eines Formenkreises von in verschiedenen Spezies vorkommenden übertragbaren Enzephalopathien. In Tieren werden darunter die bovine spongiforme Enzephalopathie beim Rind (BSE), die Scrapie (Traberkrankheit) bei Schafen, Ziegen, Hamstern und Mäusen, die chronische Auszehrung (CWD) bei bestimmten amerikanischen Hirscharten, die übertragbare Enzephalopathie bei Nerzen (TME), die feline spongiforme Enzephalopathie (FSE) bei Katzen und eine spongiforme Enzephalopathie bei Antilopen näher spezifiziert. Beim Menschen unterscheidet man vier Arten von TSE: die Creutzfeldt-Jakob-Krankheit (CJD), das Gerstman-Stäussler-Scheinker-Syndrom (GSS), die fatale familiäre Insomnie (FFI) und Kuru.

Die Diagnostik ist bei Nutztieren wegen der potentiellen Übertragbarkeit durch den Verzehr von Fleisch und Blut erkrankter Tiere von großem Interesse. So besteht z. B. der Verdacht, dass der Konsum von BSE-verseuchtem Rindfleisch eine neue Variante von CJD beim Menschen (nvCJD) verursachen kann. Im Sinne des Verbraucherschutzes und der Eindämmung der Epidemie setzen daher einige Staaten behördliche Überwachungen des Durchseuchungsgrads des Rinderbestands mit BSE durch. Zu diesem Zweck werden auf Schlachthöfen routinemäßige Kontrollen geschlachteter Rinder angeordnet, von denen die weitere Verwendung des Schlachtguts abhängt.

Eine sichere TSE-Diagnose ist bislang nur post mortem a) durch den histologischen Nachweis charakteristischer spongiformer (schwammartiger) Veränderungen im Hirngewebe, b) durch den immunologischen Nachweis von Ablagerungen des pathologischen Prionproteins (PrP) mittels Immunoblotting (Western-Blot-Technik und Histo-Blot-Technik) und Immunohistochemie, c) durch den elektronenmikroskopischen Nachweis Scrapie-assoziierter (PrP)-Fibrillen (SAF) und d) durch den Nachweis des infektiösen TSE-Agens mittels Übertragungsexperimenten im Tierversuch möglich.

Bisher sind zwei diagnostische Verfahren zur Identifizierung von TSE-infizierten Nutztieren in großtechnischem Maßstab verfügbar, der "Prionics Check" der Schweizer Fa. Prionics AG und ein Test der Fa. BIO-RAD ("Platelia BSE") . Beide Verfahren sind post mortem -Tests anhand von Gewebe aus dem Hirnstamm und auf die Anwendung im Schlachthof begrenzt. Der Prionics-Test erlaubt die Diagnose einer BSE im Rind nach Angaben des Herstellers bis zu einem halben Jahr vor dem Auftreten klinischer Symptome (Informationen des Herstellers im Internet: www.prionics.com). Bei dem von der Fa. Prionics entwickelten Verfahren wird eine Gewebeprobe aus der Medulla oblongata geschlachteter Rinder entnommen, homogenisiert und mit dem Enzym Proteinase K behandelt. Das ggf. nach der Behandlung verbleibende pathologische Prionprotein wird mit dem monoklonalen Antikörper 6H4 (hergestellt von der Firma Prionics AG) markiert und anschließend im Western-Blot angefärbt. Bei dem Platelia-Test wird eine Hirnstammprobe homogenisiert und mit Proteinase K behandelt. Anschließend wird das pathologische Prionprotein durch Fällung konzentriert, in einem Capture-ELISA mittels eines monoklonalen Antikörpers gebunden und mit Hilfe eines zweiten monoklonalen Antikörpers markiert und angefärbt.
Die Nachteile dieser Methoden bestehen in dem jeweils sehr aufwändigen Verfahren, bei dem zum Teil hochspezialisierte Fachleute erforderlich sind, um die Entnahme, Präparation und Analyse der Proben durchführen zu können, sowie in der Dauer von ca. 5 (BIO-RAD-Test) bzw. bis zu 8 (Prionics-Test) Stunden, die nach Angaben der Hersteller vom Zeitpunkt der Probenentnahme bis zum Erhalt des Resultats vergehen. Ein weiterer Nachteil der post mortem - Diagnostik ist dadurch gegeben, daß hierbei verfahrensbedingt infizierte Rinder erst nach ihrer zumindest teilweisen Zerlegung im Schlachthof identifiziert werden. Somit vermag die post mortem -Diagnostik akzidentellen Kontaminationen und Infektionen im Schlachthof nicht wirksam vorzubeugen. Dies wäre wesentlich besser zu gewährleisten, wenn infizierte Rinder ante mortem ohne Eröffnung des Körpers identifiziert und dann unschädlich beseitigt werden könnten.

Zur Zeit befinden sich verschiedene Testsysteme in der Entwicklung, um ante und post mortem ein sensitives und schnelles Screening großer Zahlen von Proben unterschiedlicher Gewebe und Körperflüssigkeiten auf pathologische Prionproteine und somit eine TSE-Diagnostik im großen Maßstab zu ermöglichen. Dazu zählen u. a. auch ein nichtevaluierter Kapillarelektrophorese-Immunassay mit fluoreszenzmarkierten Peptiden (Schmerr & Jenny: Elektrophoresis 19, (1998) 409-419; Schmerr et al.: Journal of Chromatography A 853, (1999)207-214) und ein Immunassay mit fluorometrisch detektierten, Europium-markierten Antikörpern (DELFIA, Fa. Wallac, Turku, Finnland; Safar et al.: Nature Medicine 4, (1998) 1157-1165).

Klinische Symptome und der laborchemische Nachweis erhöhter Konzentrationen bestimmter Proteine im Liquor und/oder Serum [Protein 14-3-3 (Zerr et al. (1997) N. Engl. J. Med. 336:874, Zerr et al. (1998) Ann. Neurol. 43: 572-573); Protein S100 (Otto et al. (1997) J. Neurol. 244: 566-570, Otto et al. (1998) Brit. Med. J. 316: 577-582; Otto et al. (1998) J. Neurovirol. 4: 572-573)und neuronspezifische Enolase (Zerr et al. (1995) Lancet 345: 1609-1610)] erlauben bei Menschen und Tieren lediglich eine Verdachtsdiagnose. Gleiches gilt für die veränderten Resultate, die im Zusammenhang mit menschlichen TSE-Erkrankungen bei EEG- und magnetresonanztomographischen Untersuchungen auftreten. Bei der Diagnose der BSE bei Rindern haben die post mortem und mit hohem Aufwand durchführbaren bekannten Untersuchungsverfahren unter Verwendung von Gewebeproben noch den entscheidenden Nachteil, dass die Gewebeuntersuchung erst nach der Tötung der betreffenden Tiere erfolgen kann. Das heißt, ein Screening lebender Herden oder eine frühzeitig einsetzende, in Abständen wiederholbare Untersuchung am lebenden Objekt sind bisher nicht möglich.

Aus der DE 199 23 811 C 1 ist schließlich bereits ein Verfahren für die schnelle Identifizierung von durch transmissible spongiforme Enzephalopatien induzierte pathologische Veränderungen in tierischen oder menschlichen Geweben mit Infrarotspektroskopie bekannt, bei dem Infrarotstrahlung auf eine durch TSE pathologisch veränderte Gewebeprobe gelenkt wird und die spektralen Charakteristika der Infrarotstrahlen nach Wechselwirkung mit der Gewebeprobe registriert werden und die so erhaltenen Infrarotspektren mit einer Referenzdatenbank, die Infrarotspektren von TSE-infizierten und nicht infizierten Geweben enthält, verglichen und klassifiziert werden.

Dieses Verfahren, das sich leicht automatisieren lässt, einen geringen Personal- und Materialbedarf erfordert und ohne hochspezialisierte Fachleute auskommt, ist diagnostisch hochempfindlich und liefert in kürzester Zeit sichere, von menschlichen Fehleinschätzungen freie Ergebnisse. Es ist jedoch insofern nachteilig, als seine Anwendung auf pathologisch geschädigtes Gewebe, insbesondere Gewebeproben aus dem zentralen Nervensystem, in dem nachweisbare pathologische Veränderungen ebenfalls zu erwarten sind, beschränkt ist. Derartiges Gewebematerial kann nur "post mortem", z.B. im Schlachthof bei für den Konsum vorgesehenen, getöteten Tieren, entnommen werden. Eine frühzeitig einsetzende, in Abständen wiederholbare Untersuchung lebender Tiere und deren rechtzeitige Aussonderung im Falle einer festgestellten Infektion ist mit diesem Verfahren nicht möglich. Zudem besteht bei der "post mortem"-Diagnostik im Schlachthof, bei der infizierte Tiere erst nach ihrer Zerlegung identifiziert werden, die Gefahr von Kontaminationen und Infektionen.

Der Erfindung liegt daher die Aufgabe zugrunde, ein Verfahren zum Nachweis von TSE-induzierten Veränderungen im menschlichen und tierischen Körper anzugeben, das mit geringem Aufwand im Rahmen einer frühzeitig einsetzenden, wiederholbaren Untersuchung lebender Individuen einen schnellen und zuverlässigen Nachweis von TSE-Infektionen ermöglicht.

Erfindungsgemäß wird die Aufgabe mit einem Verfahren gemäß den Merkmalen des Patentanspruches 1 gelöst.

Der wesentliche Erfindungsgedanke besteht in dem Vorschlag, eine TSE-Infektion IR-spektroskopisch in Körperflüssigkeiten nachzuweisen, die dem lebenden Individuum, beispielsweise in Form von Blut, entnommen werden können, in denen aber für die Krankheit kennzeichnende Schäden nicht manifestiert sind und die auch keine sonstigen bekannten pathologischen Schäden aufweisen. Das Wesen der Erfindung liegt mit anderen Worten in dem erstmaligen IRspektroskopischen Nachweis von durch TSE bedingten, aber von der Fachwelt dort nicht erwarteten molekularen Veränderungen in ante mortem entnehmbaren Körperflüssigkeiten oder deren Fraktionen, und der Tatsache, dass ein derartiger Vorschlag trotz der überragenden Vorteile und des lange anhaltenden dringenden Bedürfnisses bisher unterblieben ist.

Gemäß der Erfindung werden zunächst von ante mortem entnommenen Körperflüssigkeitsproben sowohl TSE-infizierter als auch gesunder Individuen Infrarotspektren angefertigt und daraus Bereiche mit charakteristischen Wellenlängen oder spektralen Merkmalen ausgewählt sowie eine entsprechende Referenzdatenbank aufgestellt. Der Nachweis von TSE bei den zu untersuchenden Individuen wird nun mit Hilfe der diesen ante mortem entnommenen Körperflüssigkeitsproben oder Körperflüssigkeitsfraktionen und den daraus erzeugten Infrarotspektren bzw. in mind. einem ausgewählten charak-teristischen Spektralbereich in der Weise geführt, dass die ausgewählten charakteristischen Spektralbereiche der aktuellen Proben mit den entsprechenden charakteristischen Spektralbereichen der Referenzproben von gesunden und auch von kranken Individuen verglichen werden. Sowohl aus dem Unterschied als auch aus der Übereinstimmung zwischen den charakteristischen Merkmalen der Referenzspektren von kranken und gesunden Individuen und den charakteristischen Merkmalen der aktuellen Untersuchungsspektren kann mit Sicherheit festgestellt werden, ob die untersuchte Körperflüssigkeit von einem gesunden oder TSE-kranken Individuum stammt. Durch den Vergleich des aktuellen Probenspektrums mit den Referenzspektren von gesunden und TSE-kranken Individuen wird eine zweifache Sicherheit bei der Beurteilung des aktuellen Probenspektrums erreicht. Es versteht sich, dass die Erstellung der Referenzspektren und der aktuellen Probenspektren hinsichtlich Probennahme, Probenpräparation und die Aufbereitung der Spektren und die Auswahl der charakteristischen Spektralbereiche unter identischen Bedingungen erfolgt. Das heißt, alle Parameter für die Referenz- und Probenmessung müssen identisch gewählt werden.

Die Vorteile der Erfindung liegen insbesondere darin, dass mit einer *in vivo* durch einen kleinen Eingriff bereitgestellten Probe in weniger als einer Minute ein in höchstem Maße sicherer Nachweis über das Vorliegen einer TSE-Infektion gelingt. Damit lässt sich auch die Differentialdiagnostik humaner TSE-Erkrankungen verbessern. Die hohe Sicherheit des Untersuchungsergebnisses ist insbesondere auch darin begründet, dass die Bewertung bzw. der Vergleich der Spektren ausschließlich anhand mathematischer Kriterien erfolgt und subjektive menschliche Fehleinschätzungen ausgeschlossen sind. Der *in vivo* durchgeführte TSE-Nachweis bietet zudem potentiell die Möglichkeit, bei der Behandlung von TSE und bei der Entwicklung von Medikamenten durch schnelle und unkomplizierte Verlaufskontrollen die Wirksamkeit der Medikamente zu prüfen. Auch Blut-, Organ- und Gewebespender können mit dem erfindungsgemäßen Verfahren mit geringem Aufwand und mit hoher Sicherheit auf TSE untersucht werden. Eine besondere Bedeutung kommt dem vorgeschlagenen Verfahren sowohl bei der Erfassung der Ursachen von BSE bei Nutztieren als auch beim Verbraucherschutz zu, da es nunmehr möglich ist, den gesamten Nutztierbestand kontinuierlich zu untersuchen. Wesentliche Vorteile liegen auch in dem gegenüber den bekannten Verfahren verringerten zeitlichen und materiellen Aufwand aufgrund der unmittelbaren IRspektroskopischen Untersuchung der Körperflüssigkeiten oder Flüssigkeitsfraktionen, die als infrarot-transparenter trockener oder flüssiger Film der Infrarotstrahlung ausgesetzt werden.

Aus den Unteransprüchen sowie der unten beispielhaft erläuterten Erprobung des vorgeschlagenen Verfahrens ergeben sich weitere Merkmale und vorteilhafte Weiterbildungen der Erfindung.

Als Probenmaterial werden vorzugsweise Blut und Fraktionen von Blut, insbesondere Blutserum, verwendet.

Zur Darstellung charakteristischer unterscheidbarer Muster der erzeugten Infrarotspektren werden eine Wellenlängenselektion und ein Algorithmus zur "feature selection" angewendet, um die geringen spektralen Unterschiede zwischen infiziertem und nicht infiziertem Probenmaterial deutlich erkennbar zu machen.

Nach einem weiteren Merkmal der Erfindung werden Multikuvetten für das Messen mehrerer Proben eingesetzt. Des weiteren können mikrospektrometrische Techniken oder bei Mikrotiterplatten benutzte Probenträger verwendet werden. Als Probenträger kommen Küvetten aus wasserunlöslichen optischen Materialien oder auch aufgerauhte Metallplatten, Metallgitter oder Durchflussküvetten in Frage. Auch der Einsatz von Infrarotlichtleitern ist denkbar. Bei geringer Probenmenge ist somit eine automatisierte Probenvorbereitung und -messung und ein hoher Probendurchsatz möglich.

In weiterer Ausbildung der Erfindung wird das Infrarotspektrum der vorbereiteten Proben im mittleren Infrarotbereich zwischen 500 und 4000 cm⁻¹ und/oder im nahen Infrarotbereich zwischen 4000 und 10.000 cm⁻¹ aufgenommen.

Nach einem weiteren Merkmal der Erfindung erfolgt die Auswahl markanter Spektralbereiche zur optimierten Unterscheidung der Spektren für die Auswertung entweder visuell oder durch multivariate Verfahren zur Selektion spektraler Merkmale. Hier hat sich zum Beispiel die Kovarianz-Analyse oder die einfache univariate VarianzAnalyse bewährt, geeignete Spektralbereiche zu finden. Aber auch andere Methoden wie die Wellenlängenselektion mittels genetischen Algorithmen und einer Kombination mit einem statistischen Kriterium, etwa einem Diskriminanzverfahren oder einem statistischen Distanzmaß, sind dazu geeignet.

Nach einem weiteren Merkmal der Erfindung erfolgt unabhängig von der Wahl des Verfahrens zur Wellenlängenselektion eine vorherige Aufbereitung der Spektren, die sich als vorteilhaft erwiesen hat. In Frage kommende Methoden dazu sind die Berechnung der ersten oder zweiten Ableitung, die Spektren-Dekonvolution oder andere Verfahren zur Erhöhung des spektralen Kontrastes, die eine Bandenerkennung erleichtern oder die Minimierung etwaiger Basislinienprobleme gestatten. Auch die Datenreduktion oder Transformation, wie etwa durch eine Wavelet-Transformation oder die Zerlegung in die Faktoren durch die Hauptkomponentenanalyse oder weitere Verfahren der multivariaten Statistik, bietet die Möglichkeit, eine vorherige Datenreduktion und Verbesserung der folgenden Klassifikation in infizierte und nicht-infizierte Tiere zu erreichen.

In weiterer Ausbildung der Erfindung erfolgt nach der Auswahl charakteristischer Spektralbereiche und nach einer vorangegangenen Aufbereitung der Spektren mit Methoden zur Erhöhung des spektralen Kontrastes und zur Datenreduktion die Klassifikation der markanten Spektralbereiche zur Feststellung von Unterschieden zwischen den Referenz- und den aktuellen Probenspektren mit statistischen Verfahren zur Mustererkennung, mit künstlichen neuronalen Netzen, Methoden des fallbasierten Klassifizierens oder des maschinellen Lernens oder mit genetischen Algorithmen oder durch evolutionäres Programmieren. Vorzugsweise wird zur Klassifikation ein künstliches neuronales Netz als feed-forward-Netz mit drei Lagen und einer Gradientenabstiegsmethode als Lernalgorithmus verwendet.

Nachfolgend wird eine in Verbindung mit dem erfindungsgemäßen Verfahren durchgeführte Versuchsreihe beschrieben: Erwachsene männliche und weibliche Syrische Hamster (Mesocricetus auratus) wurden mit dem Scrapie Stamm 263 K infiziert. Dabei wurden 27 Tiere intracerebral (i.c.), 90 Tiere intraperitoneal (i.p.) sowie 29 Tiere peroral (p.o.) infiziert. Die Anzahl der uninfizierten Kontrolltiere betrug 113. 22 Tiere wurden i.p. mock-infiziert und 31 Tiere p.o. mock-infiziert. Die Hamster der i.c. -Serie erhielten 50*µ*l Inokulum mit unterschiedlichem Gehalt an infektiösem 263 K Agens. Die i.p.- und p.o. Serie wurde mit 100*µ*l einer 10% Suspension von 263 K Scrapie Gehirnhomogenisat mit einer Dosis von 1-3*10⁷ LD₅₀ i.c., wie bei Baldauf et al. (1997, J. gen. Virol. 78, 1187-1197) beschrieben, infiziert. Den Kontrolltieren wurde entweder i.p. oder p.o. Gehirnhomogenisat aus nicht-infizierten Spendern verabreicht oder sie blieben vollkommen ohne Behandlung. Alle Rezipienten waren bei Verabreichung des Inokulums 4-6 Wochen alt. Die Inkubationszeit der i.c.-infizierten Tiere betrug 83 bis 210 Tage. Die i.p. und p.o. -infizierten Hamster hatten eine mittlere Inkubationszeit von 118 ± 12 (SD) und 157 ± 9 (SD) Tage. Alle Tiere wurden im terminalen Stadium der Scrapie-Infektion mit CO₂ euthanasiert. Die i.p. mock-, die p.o. mock- und die nicht-infizierten Tiere wurden in einem Alter von 140-180, 170-210 bzw. 30-140 Tagen getötet.
Die Entnahme von Blut und die Isolierung des Serums wurde durchgeführt wie bei Otto et al. (1998, J. Neurovirol. 4: 572-573) beschrieben.

Für die Analysen wurden jeweils 2,6 µl des Blutserums auf eine ZnSe-Multiprobenküvette transferiert, an der Luft dehydriert und in einem Trockenschrank bei 37°C für 5 Minuten zu transparenten Filmen getrocknet. Danach war die Probenpräparation abgeschlossen und die Messküvette wurde in ein FT-IR Spektrometer (IFS 28/B, Bruker Optik GmbH, Germany) überführt und die Aufnahme der FT-IR Spektren der Proben durchgeführt. Die Spektren wurden im Wellenzahlbereich von 4000 - 500 cm⁻¹ bei einer nominalen physikalischen Auflösung von 4 cm⁻¹ mit einem deuterierten Triglycinsulfat-Detektor (DTGS) aufgenommen. Als Apodisationsfunktion für die Fourier-Transformation wurde eine Blackmann-Harris 3-term Funktion und ein Zerofilling-Faktor von 4 angewendet. 128 Scans wurden aufgenommen und gemittelt. Alle Proben wurden jeweils 3-fach ggf. zu verschiedenen Zeiten gemessen.
Für die weitergehende Datenauswertung wurden die zweiten Ableitungen der Absorptions-Spektren mit einer Savitzky-Golay-Glättung von 9 Datenpunkten und einer anschließenden Vektornormalisierung im Bereich von 2820-2985cm⁻¹ durchgeführt. Die Daten besaßen dadurch vergleichbare Wertebereiche.

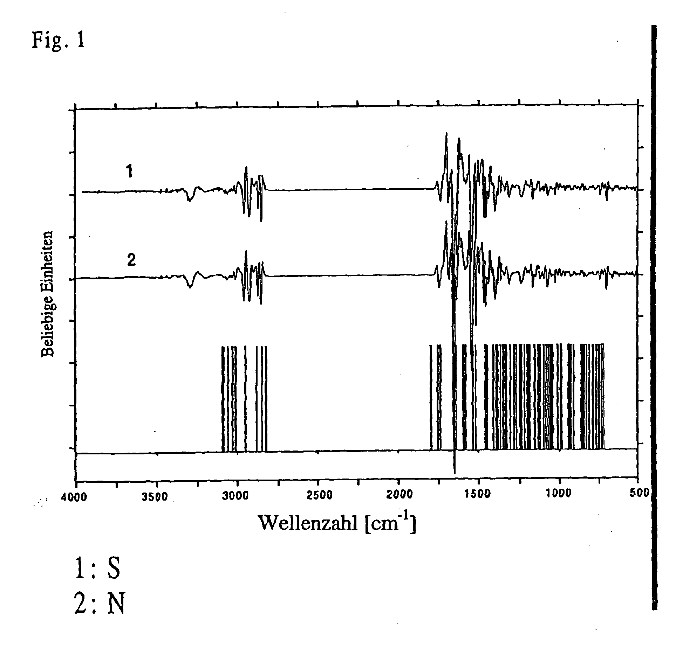

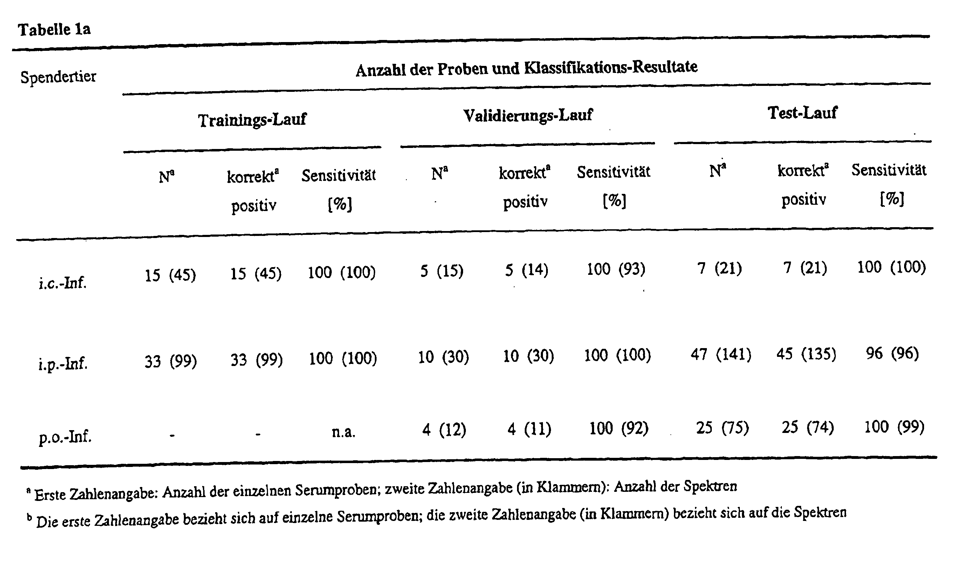

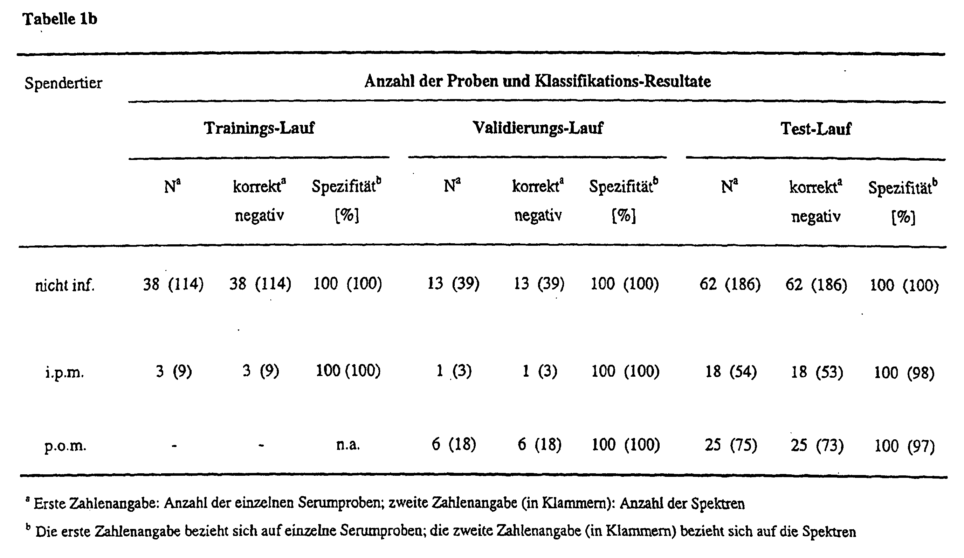

Die Messungen wurden in drei unabhängige Datensätze unterteilt: einen Trainingsdatensatz, einen Validierungsdatensatz und einen Testdatensatz. Der Trainingsdatensatz enthielt 89 Proben mit 267 Spektren, der Validierungsdatensatz 39 Proben mit 117 Spektren und einen Testdatensatz mit 184 Proben und 522 Spektren (s. Tabelle 1).
Das aufgezeichnete FT-IR Spektrum von 4000-500 cm⁻¹ zeichnete sich durch einen hohen Anteil an redundanter Information aus. Deshalb wurde zur Verbesserung des Klassifikationsmodells ein Algorithmus zur "feature selection" angewendet. Damit wird die Komplexität und die Dimensionalität des Klassifikationsmodells verringert und es werden nur noch die relevanten spektralen Informationen herangezogen. Die Güte und Robustheit des Auswertesystems konnte damit wesentlich verbessert werden. Dazu wurde zunächst eine Verringerung der Datenpunkte durch eine Mittelwertbildung über 3 Punkte durchgeführt, danach wurde in den spektralen Fenstern von 700-1500 cm⁻¹ und 1700-1750 cm⁻¹ sowie 2800-3100 cm⁻¹ die Kovarianz über den partiellen F-Wert der 2 Klassen ("infizierte" und "nicht-infizierte" Seren) über die Datenpunkte berechnet. Danach wurden eine Ordnung mit absteigender Wertereihenfolge der Kovarianzwerte aufgestellt und die 89 Wellenlängen mit den höchsten Werten für das Klassifikationsmodell herangezogen. Die spektralen Unterschiede, die sich nach der Wellenlängenselektion durch einen "feature selection"-Algorithmus in den Spektren der infizierten und in den nicht infizierten Tieren ergeben, sind in Fig. 1 dargestellt.
Fig. 1 zeigt repräsentative zweite Ableitungen der FT-IR Spektren von Hamster-Serum von (1) einem uninfiziertem Kontrolltier und (2) einem mit Scrapie i.p.-infiziertem Tier im terminalen Stadium. Die spektralen Bereiche, die nach der Berechnung der Kovarianz der zwei Klassen Scrapie-infiziert und nicht-infiziert den wichtigsten Beitrag für die Klassifikation liefern, sind als Balken dargestellt.

Als Klassifikationsmodell kam ein neuronales Netz zum Einsatz, das mit dem Synthon NeuroDeveloper (Synthon, Gusterath) erstellt wurde. Es handelte sich um ein "vorwärtsgerichtetes Netz" (feedforward net) mit 3 Lagen: einer Eingabeschicht, einer verdeckten Schicht und einer Ausgabeschicht. Die Eingabeschicht besaß 89 Neuronen (units), die verdeckte Schicht 9 Neuronen (units) und die Ausgabeschicht 2 Neuronen (units). Die Neuronen der Ausgabeschicht wurden der Klasse "infiziert" (S) und "nicht infiziert" (N) zugeordnet. Das Netz war vollständig verbunden und besaß shortcut-connections, d. h., direkte Verbindungen von der Eingabe- zur Ausgabeschicht. Als Lernverfahren wurde RPROP (resilient backproppagation) eingesetzt (Riedmiller & Braun 1993, Proc. of the IEEE Intern. Conf. on Neural Networks ICNN San Francisco, 591-598, Schmitt & Udelhoven 2000, In. H.-U. Gremlich & B. Yan, Infrared and Raman spectroscopy of biological materials, Marcel Dekker, New York, 379-420), und zwar mit einem update-Wert von 0.1 und einer Schrittweite von 50. Eine logistische Funktion wurde als Transferfunktion verwendet und die Initialisierung erfolgte im Wertebereich von [-1,+1]. 450 Trainingszyklen wurden berechnet.

Das Ergebnis dieses Auswertemodells zur Klassifikation ist in Tabelle 1a und Tabelle 1b wiedergegeben.

Es zeigen:
Tabelle la: Prüfung und Klassifikation von Serumproben von intrazerebral (i.c.), intraperitoneal (i.p.) oder oder peroral (p.o.) infizierten Scrapie-Hamstern mit FT-IR Spektroskopie und einer Analyse mit künstlichen neuronalen Netzen.
   Klassifizierung des Tieres als positiv, wenn 2 oder 3 Spektren als positiv eingestuft werden. Klassifizierung als negativ, wenn nur 1 oder kein Spektrum als positiv eingestuft wurde.
Tabelle 1b: Prüfung und Klassifikation von Serumproben von nicht infizierten, intraperitoneal mock (i.p.m.) - infizierten oder peroral mock (p.o.m.) - infizierten Kontroll-Hamstern mit FT-IR-Spektroskopie und einer Analyse mit künstlichen neuronalen Netzen.
   Klassifizierung des Tieres als positiv, wenn 2 oder 3 Spektren als positiv eingestuft werden. Klassifizierung als negativ, wenn nur 1 oder kein Spektrum als positiv eingestuft wurde.

## Patentansprüche

1. Verfahren zum Nachweis von TSE-induzierten Veränderungen im menschlichen und tierischen Körper, bei dem von bekanntermaßen mit TSE infizierten Proben sowie nicht mit TSE infizierten Proben Infrarotspektren erzeugt und charakteristische Muster von diesen in einer Referenzdatenbank gespeichert werden sowie von aktuell zu untersuchendem Probematerial ein aktuelles Infrarotspektrum erzeugt, und dessen charakteristisches spektrales Muster mit dem der Referenzdatenbank verglichen wird, wobei zur Darstellung charakteristischer unterscheidbarer Muster der Infrarotspektren eine Wellenlängenselektion und ein Algorithmus zur "feature selection" angewendet wird um die geringen spektralen Unterschiede zwischen infiziertem und nicht infiziertem Probematerial herauszustellen **dadurch gekennzeichnet, dass** das Probematerial Blutplasma oder Blutserum ist, die bereits ante mortem von dem betreffenden Körper entnommen werden kann und als infrarot-transparenter trockener oder flüssiger Film der Infrarotstrahlung ausgesetzt wird. wobei bei diesem Algorithmus zunächst eine verringerung der Datenpunkte durch Mittel wertbildung erfolgt, danach die Kovarianz über die Datenpunkte in den spektralen Fenstern von 700-1500 cm⁻¹ und 1700-1750 cm⁻¹ sowie 2800-3100 cm⁻¹ berechnet wird, danach eine Ordnung der Wellenlängen mit absteigender Wertereihenfolge der Kovarianzwerte aufgestellt und die Wellenlängen mit den höchsten Werten in ein Klassifikationsmodell eingegeben wird.

2. Verfahren nach Anspruch 1, **dadurch gekennzeichnet, dass** die Infrarotbestrahlung der Proben unter Verwendung von Multiküvetten oder Durchflussküvetten, von für Mikrotiterplatten verwendeten Probenträgern oder unter Einsatz mikrospektrometrischer Techniken durchgeführt wird.

3. Verfahren nach Anspruch 2, **dadurch gekennzeichnet, dass** ais Probenmaterial wasserunlösliche optische Materialien, aufgerauhte Metallplatten oder Metallgitter eingesetzt werden.

4. Verfahren nach einem der Ansprüche 1 bis 3, **dadurch gekennzeichnet, dass** die Durchmesser der durchstrahlten Probenareale zwischen 0,5 und 12 mm bzw. bei Mikrofokussierung zwischen 10 und 500 µm liegen.

5. Verfahren nach einem der Ansprüche 1 bis 4, **dadurch gekennzeichnet, dass** das Infrarotspektrum der Körperflüssigkeitsproben im mittleren Infrarotbereich von 500 bis 4000 cm⁻¹ gemessen wird.

6. Verfahren nach einem der Ansprüche 1 bis 5, **dadurch gekennzeichnet, dass** die Erzeugung und Messung des Infrarotspektrums in einer Anordnung mit Transmission/Absorption oder abgeschwächter Totalreflexion oder direkter bzw. diffuser Reflexion oder mit IR-Lichtleitertechnik durchgeführt wird.

7. Verfahren nach einem der Ansprüche 1 bis 6, **dadurch gekennzeichnet, dass** eine Vorverarbeitung der Spektren durch die Bildung der ersten oder zweiten Ableitung, durch Spektren-Dekonvolution oder andere Verfahren zur Erhöhung des spektralen Kontrastes vorgenommen wird, oder durch Transformation wie etwa die Wavelet-Transformation oder die Transformation in Hauptkomponenten durch die Hauptkomponentenanalyse erfolgt.

8. Verfahren nach einem der Ansprüche 1 bis 7, **dadurch gekennzeichnet, dass** die Auswahl der TSE-spezifischen charakteristischen Spektralbereiche visuell oder nach computergestützten Methoden oder Wellenlängenselektion durchgeführt wird.

9. Verfahren nach Anspruch 8, **dadurch gekennzeichnet, dass** die Erkennung und Auswahl der charakteristischen Spektralbereiche mit Hilfe von genetischen Algorithmen, statistischen Distanzmaßen, der Kovarianz- und univariaten Varianzanalyse oder durch mathematische Transformation, oder einer Kombination von genetischen Algorithmen und statistischen Methoden wie der Diskriminanzanalyse oder der Hauptkomponentenanalyse, erfolgt.

10. Verfahren nach einem der Ansprüche 1 bis 9, **dadurch gekennzeichnet, dass** die Klassifikation bzw. der Vergleich des aktuellen Infrarotspektrums in den ausgewählten, charakteristischen Spektralbereichen mit den Referenzspektren in den entsprechenden Spektralbereichen nach statistischen Merkmalen der Mustererkennung oder auf der Grundlage neuronaler Netze, des maschinellen Lernens oder von Algorithmen der mulivariaten Statistik vorgenommen wird.

11. Verfahren nach Anspruch 10, **dadurch gekennzeichnet, dass** die Klassifikation auf der Grundlage eines künstlichen neuronalen Netzes als feed-forward-Netz mit drei Lagen und einer Gradientenabstiegsmethode als Lernalgorithmus durchgeführt wird.

## Claims

1. Method of detecting TSE-induced changes in the human and animal body, wherein infrared spectra of samples known to be infected with TSE and of samples not infected with TSE are generated and characteristic patterns thereof are stored in a reference data bank, and an infrared spectrum of sample material currently being investigated is generated and the characteristic spectral pattern thereof is compared with that of the reference data bank, wherein for displaying characteristic distinguishable patterns of the infrared spectra there is used a wavelength selection and an algorithm for "feature selection" in order to reveal the very small spectral differences between infected and non-infected sample material, **characterised in that** the sample material is blood plasma or blood serum which can be taken from the body in question ante-mortem and which in the form of an infrared-transparent dry or liquid film is exposed to infrared radiation, wherein in this algorithm the number of data points is first reduced by averaging, then the covariance over the data points in the spectral windows of 700-1500 cm⁻¹ and 1700-1750 cm⁻¹ and also 2800-3100 cm⁻¹ is calculated, the wavelengths are then ranked in descending order of the covariance values and the wavelengths having the highest values are entered into a classification model.

2. Method according to claim 1, **characterised in that** the infrared irradiation of the samples is carried out using multicuvettes or flowthrough cuvettes, using sample carriers employed for microtitre plates or using microspectrometric techniques.

3. Method according to claim 2, **characterised in that** water-insoluble optical materials, surface-roughened metal plates or metal grids are used as sample carrier material.

4. Method according to any one of claims 1 to 3, **characterised in that** the diameters of the sample areas through which radiation is passed are from 0.5 to 12 mm or, in the case of microfocussing, from 10 to 500 µm.

5. Method according to any one of claims 1 to 4, **characterised in that** the infrared spectrum of the body fluid samples is measured in the mid-infrared range of from 500 to 4000 cm⁻¹.

6. Method according to any one of claims 1 to 5, **characterised in that** the generation and measurement of the infrared spectrum is carried out in an arrangement having transmission/absorption or attenuated total reflection or direct or diffuse reflection or with IR light conductor techniques.

7. Method according to any one of claims 1 to 6, **characterised in that** preprocessing of the spectra is effected by the formation of the first or second derivative, by spectra deconvolution or other methods of increasing the spectral contrast, or is effected by transformation, such as, for example, wavelet transformation or transformation into principal components by principal component analysis.

8. Method according to any one of claims 1 to 7, **characterised in that** the selection of the TSE-specific characteristic spectral regions is carried out visually or by computer-aided methods or wavelength selection.

9. Method according to claim 8, **characterised in that** the recognition and selection of the characteristic spectral regions is effected with the aid of genetic algorithms, statistical distance measurements, covariance and univariate variance analysis or by mathematical transformation, or a combination of genetic algorithms and statistical methods, such as discriminance analysis or principal component analysis.

10. Method according to any one of claims 1 to 9, **characterised in that** the classification or comparison of the current infrared spectrum in the selected, characteristic spectral regions with the reference spectra in the corresponding spectral regions is carried out in accordance with statistical features of pattern recognition or on the basis of neural networks, machine learning or algorithms of multivariate statistics.

11. Method according to claim 10, **characterised in that** the classification is carried out on the basis of an artificial neural network as feed-forward network with three layers and a gradient descent method as learning algorithm.

## Revendications

1. Procédé de détection des modifications induites par l'encéphalopathie spongiforme transmissible dans le corps humain et le corps animal, dans lequel sont produits de façon connue des spectres infrarouges par des échantillons infectés par l'encéphalopathie spongiforme transmissible et par des échantillons non infectés par l'encéphalopathie spongiforme transmissible, et des modèles caractéristiques de ceux-ci sont stockés dans une base de données de référence, et un spectre infrarouge actuel est produit par un échantillon de matériau actuel devant être analysé, et dont le modèle spectral caractéristique est comparé à celui de la base de données de référence, moyennant quoi en vue de la représentation de modèles distinctifs caractéristiques des spectres infrarouges, une sélection de longueurs d'ondes et un algorithme de sélection de caractéristique (« feature selection ») est appliqué en vue de faire apparaître les faibles différences spectrales entre l'échantillon de matériau infecté et non infecté, **caractérisé en ce que** l'échantillon de matériau est du plasma sanguin ou du sérum sanguin, qui peut être déjà prélevé avant le décès sur le corps concerné et est exposé en tant que film sec ou liquide transparent aux infrarouges aux rayons X, moyennant quoi dans cet algorithme, une réduction des points d'informations a tout d'abord lieu par la formation de moyennes, puis la covariance est calculée par les points d'informations dans les fenêtres spectrales de 700-1500 cm⁻¹ et 1700-1750 cm⁻¹, et 2800-3100 cm⁻¹, puis un classement des longueurs d'ondes est établi avec le séquencement décroissant des valeurs de covariance et les longueurs d'ondes ayant les valeurs les plus élevées sont entrées dans un modèle de classification.

2. Procédé selon la revendication 1, **caractérisé en ce que** le rayonnement infrarouge des échantillons est réalisé en utilisant des cuvettes multiples ou des cuvettes de circulation, des supports d'échantillons utilisés pour des plaques microtitrées ou en utilisant des techniques microspectrométriques.

3. Procédé selon la revendication 2, **caractérisé en ce que** des matériaux optiques insolubles dans l'eau, des plaques métalliques brossées ou des grilles métalliques sont utilisés en tant qu'échantillons de matériau.

4. Procédé selon une des revendications 1 à 3, **caractérisé en ce que** les diamètres des zones d'échantillons radiographiées sont compris entre 0,5 et 12 mm, et/ou lors de la microfocalisation entre 10 et 500 µm.

5. Procédé selon une des revendications 1 à 4, **caractérisé en ce que** le spectre infrarouge des échantillons du liquide corporel est mesuré dans la zone infrarouge moyenne de 500 à 4000 cm⁻¹.

6. Procédé selon une des revendications 1 à 5, **caractérisé en ce que** la production et la mesure du spectre infrarouge sont réalisées dans un arrangement avec la transmission/l'absorption ou la réflexion totale affaiblie ou la réflexion directe et/ou diffuse ou la technique des guides d'ondes optiques infrarouges.

7. Procédé selon une des revendications 1 à 6, **caractérisé en ce qu'**un prétraitement des spectres est assuré par la formation de la première ou de la seconde dérivation, par la déconvolution des spectres ou d'autres procédés d'augmentation du contraste spectral, ou a lieu par la transformation telle que la transformée en ondelettes ou la transformation dans les composantes principales par l'analyse des composantes principales.

8. Procédé selon une des revendications 1 à 7, **caractérisé en ce que** la sélection des régions spectrales caractéristiques spécifiques à l'encéphalopathie spongiforme transmissible est réalisée visuellement ou selon des méthodes assistées par ordinateur ou par la sélection de longueurs d'ondes.

9. Procédé selon la revendication 8, **caractérisé en ce que** l'identification et la sélection des régions spectrales caractéristiques a lieu à l'aide d'algorithmes génétiques, de mesures de distance statistiques, d'analyse de la covariance et de la variance univariante ou par la transformation mathématique, ou une combinaison des algorithmes génétiques et des méthodes statistiques telles que l'analyse discriminante ou l'analyse des composantes principales.

10. Procédé selon une des revendications 1 à 9, **caractérisé en ce que** la classification et/ou la comparaison du spectre infrarouge actuel est entreprise dans les régions spectrales caractéristiques sélectionnées avec les spectres de référence dans les régions spectrales correspondantes selon les caractéristiques statistiques de l'identification des modèles ou sur la base de réseaux neuronaux, de l'apprentissage mécanique ou des algorithmes de la statistique multivariante.

11. Procédé selon la revendication 10, **caractérisé en ce que** la classification est réalisée sur la base d'un réseau neuronal artificiel en tant que réseau de neurones à anticipation (feedforward) avec trois positions et une méthode de descente de gradient en tant qu'algorithme d'apprentissage.
